(19) **Europäisches Patentamt**

**European Patent Office**

**Office européen des brevets**

(11) **EP 1 197 219 B1**

(12) **EUROPÄISCHE PATENTSCHRIFT**

(45) Veröffentlichungstag und Bekanntmachung des Hinweises auf die Patenterteilung:
**28.12.2005 Patentblatt 2005/52**

(51) Int Cl.[7]: **A61K 35/78**

(21) Anmeldenummer: **01710050.4**

(22) Anmeldetag: **12.10.2001**

(54) **Verfahren zur Herstellung von Johanniskrautöl**

Process for the preparation of oil of St. John's wort

Procédé de préparation d'huile de millepertuis

(84) Benannte Vertragsstaaten:
**AT BE CH CY DE DK ES FI FR GB GR IE IT LI LU MC NL PT SE TR**

(30) Priorität: **12.10.2000 DE 10050574**

(43) Veröffentlichungstag der Anmeldung:
**17.04.2002 Patentblatt 2002/16**

(73) Patentinhaber: **Jukunda Naturarzneimittel Dr. Ludwig Schmitt GmbH & Co. KG**
**82142 Planegg (DE)**

(72) Erfinder:
• **Der Erfinder hat auf seine Nennung verzichtet.**

(74) Vertreter: **Groening, Hans Wilhelm**
**BOEHMERT & BOEHMERT**
**Pettenkoferstrasse 20-22**
**80336 München (DE)**

(56) Entgegenhaltungen:
**DE-A- 2 406 452**

• **DATABASE BIOSIS [Online] BIOSCIENCES INFORMATION SERVICE, PHILADELPHIA, PA, US; März 2000 (2000-03) KULEVANOVA SVETLAN ET AL: "Determination of total flavonoids and quercetin in Hyperici herba and its aqueous, aqueous-ethanolic and oil extracts." Database accession no. PREV200000292403 XP002187733 & ACTA PHARMACEUTICA (ZAGREB), Bd. 50, Nr. 1, März 2000 (2000-03), Seiten 29-37, ISSN: 1330-0075**
• **DATABASE WPI Section Ch, Week 199333 Derwent Publications Ltd., London, GB; Class B04, AN 1993-263103 XP002187734 & SU 1 752 395 A (BASHKIR MED INST), 7. August 1992 (1992-08-07)**

**Beschreibung**

Die Erfindung betrifft ein Verfahren zur Herstellung von Johanniskrautöl, bei dem Johanniskrautblüten mit einem Öl extrahiert werden.

Johanniskraut ist seit langer Zeit als medizinisch wirksame Heilpflanze bekannt. Es wird zur Behandlung von leichten Depressionen, psychovegetativen Störungen, nervösen Spannungszuständen, bei Verbrennungen, Myalgien, zur Wundbehandlung, bei Magen-, Leber-, Gallen- und Blasenbeschwerden, sowie bei Rheuma, Gicht und Kopfschmerzen eingesetzt. Insbesondere die leicht-antidepressive Wirkung konnte medizinisch erhärtet werden.

Johanniskraut (Hypericum perforatum L.) gehört zur Familie der Guttiferae. Neben Hypericin, von dem angenommen wird, daß es der wirksame Inhaltsstoff ist, enthält die Pflanze außerdem α-Pinen, Monoterpene und n-Alkane. In den goldgelben Blütenständen sind vor allem die Flavonoide Quercetin, Hyperin, Rutin, Quercitrin und Isoquercitrin enthalten. Der Gesamtgehalt an etherischen Ölen in der Pflanze beträgt ungefähr 1 %.

Als medizinisch wirksame Darreichungsformen werden wäßrige (Tees), alkoholische sowie ölige Auszüge verwendet. Feste Formen, wie Dragees, Kapseln, Filmtabletten und Tabletten können auch die getrocknete Droge in Form von Blütenbestandteilen, Blüten oder grünen Blättern enthalten. Eine weiterverbreitete Anwendung hat das sogenannte Johanniskrautöl gefunden, das einen öligen Extrakt aus den frischen Blüten der Pflanze darstellt. Das Johanniskrautöl ist ein roter, klarer Extrakt, dessen intensive Farbe durch Hypericin verursacht wird, wobei der Gehalt des Hypericins stark in Abhängigkeit von den verwendeten Pflanzenbestandteilen, der Art des Öles, dem Alter und der Herkunft der Pflanzen sowie dem Herstellungsverfahren abhängt. Das Johanniskrautöl, das auch als "Rotöl" bezeichnet wird, wird äußerlich und innerlich angewendet, in Abhängigkeit von dem angestrebten Erfolg.

Die schweizerische Patentschrift CH 680570 beschreibt ein Verfahren zur Herstellung allergenfreier Johanniskrautölextrakte, bei dem das mechanisch zerkleinerte Johanniskraut zusammen mit mechanisch zerkleinerter Kamille zusammen mit einem pflanzlichen Öl mazeriert werden, wobei das Gewichtsverhältnis von Johanniskraut zu Kamille im Bereich von 1:20 bis 20:1 liegt und wobei das Verhältnis Pflanzen:Öl 1:1 bis 1:50 beträgt. Die Extraktion findet bei Raumtemperatur für 24 Stunden unter starkem Rühren statt. Das Öl wird durch Filtern über eine Filtrationsanlage von den festen Bestandteilen getrennt und als Arzneimittel verwendet. Als pflanzliche Öle nennt diese Druckschrift Weizenkeimöl, Distelöl, Maiskeimöl, Palmkernöl, Sojaöl, Sonnenblumenkernöl, Rapsöl und Erdnußöl. In der deutschen Offenlegungsschrift 2406452 wird ein Verfahren zur Herstellung einer Salbe beschrieben, in der ein öliger Auszug von Johanniskrautblüten verwendet wird, wobei das Öl Olivenöl ist. Dabei werden, bezogen auf das Ölgewicht, 5 bis 10 % frische Johanniskrautblüten in das Öl eingebracht und in einem hermetisch geschlossenen Gefäß bei Raumtemperatur für mindestens 10 Tage ziehen gelassen. Anschließend werden die übrigen Salbenbestandteile zugefügt, um auf diese Weise die fertige Salbe zu erhalten.

Die Offenlegungsschrift DE 197 12 729 offenbart ein Heilmittel zur Anwendung bei Verletzungen, Infektionen, Allergien, Insektenstichen oder dergleichen bei Menschen und Tieren, das Weizenkeimöl umfaßt, in das Pflanzenbestandteile von Johanniskraut eingelegt wurden. In dem in der Beschreibung enthaltenen Beispiel werden 50 g bis 200 g Frischblüten von Johanniskraut auf ca. 1 Liter Weizenkeimöl bei Zimmertemperatur angesetzt, und dieser Ansatz wird einige Wochen, z. B. vier Wochen, unter Lichtausschluß gelagert.

Die europäische Patentschrift EP 01170905 offenbart ein Mittel zur Mundpflege, indem unter anderem Johanniskrautöl eingeschlossen ist, wobei eine genaue Anweisung zur Herstellung des Johanniskrautöls fehlt.

Die europäische Patentschrift EP 0 415 327 offenbart ein vasodilatierendes Mittel, in dem das Pflanzenrohmaterial von Hypericum erectum thunb mit verschiedenen organischen und anorganischen Lösungsmitteln extrahiert wird. Die Beschreibung nennt unter anderem mineralische, pflanzliche oder tierische Öle, z. B. flüssiges Paraffin, Sojaöl, Sesamöl sowie Mixturen dieser mineralischen, pflanzlichen oder tierischen Öle.

EP 0 985 416 A2 offenbart ein Verfahren und eine Vorrichtung zur Extraktherstellung aus biologischem Material, unter anderem zur Herstellung von Extrakten aus Johanniskraut. Dabei findet während der Extraktion mit polaren und/oder apolaren Lösungsmitteln eine gleichzeitige Behandlung des Extraktionsgutes mit Mikrowellen und UV-Bestrahlung statt.

Mel'nikova et al. (Pharmaceutical Chemistry Journal, Bd. 33, Nr. 12, 1999, S. 654-657) offenbart die Extraktion von einer Mischung von Blättern, Blüten und Früchten von Johanniskraut, in dem das Pflanzenmaterial zunächst in einer Wasser-Ethanolmischung vorgequollen und anschließend einer Extraktion unter Hitzeeinwirkung mittels Öl unterzogen wird. Die Autoren ziehen den Schluß, daß eine Erhöhung des relativen Volumens der wäßrigen Alkoholfraktion bei der Quellung zu einer erhöhten Extraktionseffizienz bei der anschließenden Ölextraktion führt.

Dem vorgenannten Stand der Technik ist der Nachteil zu eigen, daß die so erhaltenen öligen Extrakte durch erhebliche Schwankungen bezüglich des Hypericingehaltes des jeweils erhaltenen Rotöls charakterisiert sind, daß das Problem der Reproduzierbarkeit nicht dauerhaft gelöst worden ist und daß zudem, sofern der Extraktionsvorgang unter Bestrahlung stattfindet, die effiziente Extraktion auf Sonnenlicht angewiesen ist.

Ein weiterer Nachteil des Standes der Technik ist es, daß sich die so erhaltenen öligen Extrakte durch einen extrem strengen Geschmack und leichte Verderblichkeit auszeichnen.

Demzufolge war es Aufgabe der vorliegenden Erfindung, ein Verfahren bereitzustellen, das mit einer hohen Zuverlässigkeit erlaubt, Rotöl unabhängig von äußeren Umweltfaktoren und mit gleichbleibender Qualität herzustellen. Eine weitere Aufgabe der Erfindung war es, ein Rotöl bereitzustellen, das sich von hoher Qualität und Güte, besseren geschmacklichen Eigenschaften und einer deutlich verbesserten Haltbarkeit gegenüber nach dem Stand der Technik hergestellten Rotöl auszeichnet.

Die vorliegende Aufgabe wird gelöst durch ein Verfahren zur Herstellung von Johanniskrautöl, bei dem Johanniskrautblüten mit einem Öl extrahiert werden, gekennzeichnet durch die folgenden Schritte:

a) Vorbehandlung der Johanniskrautblüten, wobei die Vorbehandlung

aa) ein Trocknen,

ab) ein Befeuchten mit wäßriger alkoholischen Lösung umfaßt, wobei das Verhältnis des Gewichts der wäßrigen alkoholischen Lösung zum Trockengewicht der Johanniskrautblüten im Bereich von 3 : 1 - 1 : 1 liegt.

b) Extraktion der Johanniskrautblüten in einem Ansatz mit mindestens einem Öl unter Rühren und/oder Schütteln, wobei die Extraktion in Schritt b) unter kontinuierlicher künstlicher Bestrahlung erfolgt.

Die Aufgabe wird außerdem gelöst durch ein Johanniskrautöl, das durch ein Verfahren gemäß der vorliegenden Erfindung herstellbar ist, wobei als Öl zur Extraktion Sojaöl verwendet wird.

Außerdem wird die Aufgabe gelöst durch ein Johanniskrautöl, das durch mindestens eine der folgenden Eigenschaften gekennzeichnet ist:

a) eine Peroxidzahl, die im Bereich von 3-8 liegt,

b) eine deutlich reduzierte Gefahr der Ranzilität.

Bevorzugt erfolgt das Trocknen der Johanniskrautblüten bei 12 - 25°C und bevorzugter bei 15 - 20°C.

In einer Ausführungsform ist die wäßrige alkoholische Lösung, die beim Befeuchten verwendet wird, eine wäßrige ethanolische Lösung.

In einer Ausführungsform wird mindestens ein pflanzliches Öl verwendet, das ausgewählt ist aus der Gruppe, die umfaßt: Sojaöl, Weizenkeimöl, Olivenöl, Sonnenblumenkernöl, Rapsöl, Leinöl, Kürbiskemöl, Sesamöl, Maiskeimöl, Palmkernöl, Distelöl und Erdnußöl.

In einer besonders bevorzugten Ausführungsform wird Sojaöl verwendet.

In einer Ausführungsform erfolgt das Befeuchten für 15 min - 10 h, bevorzugt für mindestens 3 h, bevorzugter für mindestens 5 h.

In einer Ausführungsform des erfindungsgemäßen Verfahrens erfolgt die kontinuierliche künstliche Bestrahlung im Spektralbereich von 400 nm - 710 nm.

Bevorzugt erfolgt die kontinuierliche künstliche Bestrahlung mit einer Lichtstromstärke von 800 Lumen - 2000 Lumen, bevorzugter mit einer Lichtstromstärke von 1200 Lumen - 1600 Lumen und am bevorzugtesten mit einer Lichtstromstärke von 1350 Lumen - 1450 Lumen.

In einer Ausführungsform erfolgt die Extraktion bei einer Temperatur im Bereich von 10°C - 50°C, bevorzugt im Bereich von 20°C - 45°C und am bevorzugtesten im Bereich von 36°C - 41°C.

In einer Ausführungsform erfolgt die Extraktion für 4 - 24 Tage, bevorzugt für 8 - 20 Tage und am bevorzugtesten für 12 - 16 Tage.

Es wird bevorzugt, daß das Verfahren durch den weiteren Schritt gekennzeichnet ist:

c) Aufbereiten des Ansatzes, wobei das Aufbereiten des Ansatzes die folgenden Teilschritte umfaßt.

ca) Zugabe eines Trockenmittels unter Rühren und/oder Schütteln,

cc) Filtrieren.

Bevorzugt ist dabei das Verfahren gekennzeichnet durch den weiteren Teilschritt:

cb) Rühren und/oder Schütteln des Ansatzes.

In einer Ausführungsform ist das Trockenmittel ausgewählt aus der Gruppe, die Natriumsulfat, Calciumoxid, Kaliumcarbonat, Magnesiumsulfat und Calciumchlorid umfaßt.

In einer Ausführungsform erfolgt das Rühren und/oder Schütteln in cb) in Intervallen, mit Intervallpausen der Länge im Bereich von 15 min - 80 min.

In einer Ausführungsform des erfindungsgemäßen Verfahrens wird zur Vorbehandlung in Schritt a) eine 35% (vol/vol) - 45% (vol/vol) wäßrige alkoholische Lösung eingesetzt.

Bevorzugt liegt das Verhältnis des Gewichts der wäßrigen alkoholischen Lösung zum Trokkengewicht der Johanniskrautblüten im Bereich von 2 : 1 - 1,5 : 1.

Bevorzugt liegt das Verhältnis des Gewichts des Öls zum Trockengewicht der Johanniskrautblüten im Bereich von 22 : 1 bis 42 : 1, bevorzugter im Bereich von 30 : 1 bis 34 : 1.

In einer Ausführungsform liegt das Gewichtsverhältnis Öl zu Trockenmittel im Bereich von 100:1-150:1.

Eine Ausführungsform des erfindungsgemäß herstellbaren Johanniskrautöls weist eine Peroxidzahl auf, die im Bereich von 3-8 liegt.

Eine Ausführungsform des erfindungsgemäß herstellbaren Johanniskrautöls weist eine deutlich reduzierte Gefahr der Ranzilität auf.

Überraschenderweise hat sich gezeigt, daß mit dem erfindungsgemäßen Verfahren ein Öl von gleichbleibender, hoher Qualität hergestellt werden kann, das sich durch einen angenehmen, nicht-beißenden Eigengeschmack gegenüber herkömmlich hergestellten Öl auszeichnet. Außerdem ist es deutlich länger haltbar als ein herkömmlich hergestelltes Öl und weist im Gegensatz zu diesem einen geringeren POZ-Wert auf. In diesem Sinne ist auch der Begriff "deutlich reduzierte Gefahr der Ranzilität" verwendet, der einen Zustand der relativen Konstanz niedriger POZ-Werte bzw. eines nicht-ranzigen Geschmacks kennzeichnet. Der POZ-Wert (Peroxid-Zahl) ist ein Maß für die in Fetten und Ölen enthaltene Menge aktiven Sauerstoffs (Anzahl mEq pro 1 Kg), der in einem Gemisch aus Chloroform und Eisessig aus Kaliumjodid elementares Jod freisetzt. Die Peroxide sind verantwortlich für die "Abbaufähigkeit" von Fetten und Ölen, das heißt, je höher der Wert, desto leichter zersetzt sich das Öl, es wird ranzig. Die im Fett enthaltenen Fettsäuren zerfallen unter Aufbrechen der in den ungesättigten Fettsäuren vorhandenen Doppelbindungen in kleinere Bausteine, wie z. B. Buttersäure, etc., die für den ranzigen Geschmack eines nach dem herkömmlichen Verfahren hergestellten Öls verantwortlich ist. Überraschenderweise hat sich gezeigt, daß das Öl, das nach dem erfindungsgemäßen Verfahren hergestellt wird, einen äußerst geringen POZ-Wert aufweist und sich durch die Abwesenheit sämtlicher obengenannter unangenehmer Eigenschaften eines herkömmlichen Rotöls auszeichnet.

Die Erfindung wird nunmehr anhand der folgenden Beispiele beschrieben.

### Vergleichsbeispiel

Nach dem im Stand der Technik bisher üblichen Verfahren wird Johanniskraut, d. h. Blüten und blättrige Bestandteile geerntet, direkt nach der Ernte in großen Glasballons in Öl eingelegt, an die Sonne gestellt und für 3-4 Wochen unter täglich stattfindenden Rühren stehen gelassen. Anschließend wird das Öl gefiltert, um die flüssigen von den festen Bestandteilen zu trennen, und in Flaschen abgefüllt. Das so erhaltene Rotöl wurde auf seine Peroxidzahl untersucht. Dabei ergaben sich folgende Werte:

| Charge | POZ |
|--------|------|
| A | 12,0 |
| B | 13,8 |
| C | 13,7 |
| D | 9,95 |
| E | 12,99 |

Der durchschnittliche POZ-Wert betrug 12,49.

Aus der Tabelle wird deutlich, daß die so hergestellten Rotöle einen POZ-Wert von mindestens 9 aufweisen. Gleichzeitig haben die derart hergestellten Öle einen äußerst strengen, unangenehmen Geschmack.

Nach ca. 1 Jahr ist das Öl nicht mehr zu verwenden, was sich in einem extrem angestiegenen POZ-Wert und dem Vorhandensein von unangenehmen Abbauprodukten äußert, z. B. Buttersäure, Essigsäure, etc...

### Beispiel 1

Die Extraktion erfolgt in einem beheizbaren Edelstahl-Behälter mit Doppelmantel zum Beheizen mit Heißwasser für drucklosen Betrieb und mit einem Fassungsvermögen von ca. 3300 Liter. Zentral angeordnet in dem Behälter ist ein Glaszylinder, der das eigentliche Reaktionsgefäß darstellt, um den herum 12 Leuchtstoffröhren in regelmäßigen Abständen axial angebracht sind, die sich über die gesamte Länge des Glaszylinders erstrecken. Als Leuchtstoffröhren werden Lampen vom Typ FLUORA® L36W77 verwendet, die ein das natürliche Sonnenlicht imitierendes Spektrum haben, mit zwei Maxima, eines im Bereich zwischen 440 und 470 nm und das andere zwischen 650 und 680 nm. Die Nennleistung jeder einzelnen Leuchtstoffröhre beträgt 36 Watt, und jede Röhre hat einen Lichtstrom von 1400 lm.

**Beispiel 2**

In der in Beispiel 1 beschriebenen Anlage wurde Johanniskrautöl gemäß den folgenden Spezifikationen hergestellt.

A. Herstellung des öligen Extraktes

**Ansatzmenge 2.700 l**

| Rohstoff | Spezifikation | Menge |
|---|---|---|
| 1) Johanniskrautblüten, getrocknet | DAC 1998 | 76,5 kg |
| 2) Gereinigtes Wasser | Ph.Eur. 1997 | 90 l |
| 3) Ethanol 96 % | DAB 1997 | 63 l |
| 4) Sojaöl | Ph.Eur. 1997 | 2.700 l |
| 5) Natriumsulfat wasserfrei | Ph.Eur. 1997 | 20,0 kg |

**Vorbereitung der Droge zur Extraktion**

1. Johanniskrautblüten abwiegen und bereitstellen.

2. 90 l gereinigtes Wasser und 63 1 Ethanol 96 % (V/V) abmessen und mischen.

3. Johanniskrautblüten schichtweise in den Ansatzbehälter geben und mit dem Alkohol - Wassergemisch befeuchten.

4. Diesen Ansatz mind. 5 Stunden zugedeckt quellen lassen.

**Extraktion**

5. Die vorbereiteten Johanniskrautblüten in den in Beispiel 1 beschriebenen Behälter füllen.

6. 2.700 1 Sojaöl dazugeben und Rührwerk einschalten.

7. Beleuchtung einschalten.

8. Heizung einschalten. Temperatur auf 36 - 41°C einstellen.

9. Ansatz für 14 Tage im Behälter belassen.

10. Am 14. Tag spektrophotometrische Qualitätsbestimmung: Farbintensität entspricht Hypericinbestimmung

B. Aufbereitung des Extraktes

11. Entspricht die Farbintensität dem in der Spezifikation für das Fertigarzneimittel genannten Wert wird das Rotöl in den Vorratsbehälter gepumpt. Dabei sollten für die folgenden Parameter die folgenden Spezifikationen erfüllt werden:

| | |
|---|---|
| Aussehen | rot fluoreszierend, ölig, fast klar |
| Geschmack | charakteristisch |
| Geruch | charakteristisch |
| Brechungsindex | 1,4745-1,4755 |
| Dichte | 0,90-0,92 g pro ml |
| Peroxidzahl | 0-30 |

NB.: Mit dem neuen erfindungsgemäßen Verfahren wurden keine POZ-Werte > 7 gefunden (siehe unten).

12. 20,0 kg Natriumsulfat abwiegen.

13. Natriumsulfat zum Rotöl in den Vorratsbehälter einstreuen und Rührwerk einschalten, ½ Stunde rühren. Intervallschaltung einstellen.

14. 1 Tag intervallrühren.

15. Rotöl über Plattenfilter filtrieren bis ein klares Produkt vorliegt und in Edelstahllagertanks pumpen.

16. Filtratausbeute bestimmen.

17. Brechungsindex, Dichte, Säurezahl, Peroxid-Zahl (= POZ), Dünnschicht-Chromatographie (= DC), Hypericin.

18. Wird die Bulkware vor Abfüllung länger gelagert oder ist ein Lagertank nur teilweise befüllt, kann Stickstoff eingeleitet werden.

19. Entsprechen die Ist-Werte der gültigen Spezifikation für das Fertigarzneimittel (s.o. 11), kann das Rotöl zur Abfüllung freigegeben werden.

Das so erhaltene Johanniskrautöl ist von gleichbleibender Qualität mit einer geringen Peroxidzahl (POZ) und zeichnet sich durch eine deutlich reduzierte Gefahr der Ranzilität aus, die das Öl mit einer guten Lagerfähigkeit ausstattet, ohne daß es zu Qualitätseinbußen, insbesondere hinsichtlich der unter 11. genannten Parameter, kommt.

Für verschiedene Chargen des mit dem erfindungsgemäßen Verfahren hergestellten Rotöls ergaben sich folgende POZ-Werte:

| Charge | POZ |
|---|---|
| F | 6,9 |
| G | 6,0 |
| H | 5,0 |
| I | 7,0 |
| K | 7,0 |
| L | 3,3 |
| M | 5,5 |
| N | 3,6 |
| O | 5,6 |
| P | 6,3 |
| Q | 4,0 |
| R | 7,0 |

Der durchschnittliche POZ-Wert betrug 5,6 und liegt dabei deutlich (um 55%) unter dem oben im Vergleichsbeispiel gefundenen Mittelwert von 12,49. Das so hergestellte Öl (d. h. nach dem erfindungsgemäßen Verfahren) ist lange haltbar und weist einen konstant angenehmen, nicht beißenden Eigengeschmack auf.

**Beispiel 3**

Nach dem erfindungsgemäßen Verfahren wurden mit Sojaöl bzw. Olivenöl jeweils sechs Ansätze Johanniskrautöl hergestellt und danach eine im DAC 1986 (Deutscher Arzneimittel-Codex 1986) angegebene photometrische Methode zur Messung der Intensität der Rotfärbung durchgeführt, als Maß für das Extraktionsvermögen des jeweiligen Öles, bei der die Extinktion des Öls bei 590 mm in einer Schichtdicke von 1,000 cm gegen Methanol gemessen wird.
Unter den angegebenen Versuchsbedingungen ist die spezifische Extinktion für Hypericin $E^{1\%}_{1cm}$=718. Dabei sind die so ermittelten Prozentwerte lediglich als relatives Maß für die Intensität der Rotfärbung anzusehen und stellen keine absoluten Gewichts- oder Volumenprozentangaben dar. Sie bieten jedoch auf hervorragende und kostensparende Weise die Möglichkeit, die Rotfärbung verschiedener Öle schnell zu ermitteln und zu vergleichen. Dabei lassen sich folgende Ergebnisse feststellen:

Johanniskrautöl/**Auszugsmittel Sojaöl** - % Hypericin (Intensität Rotfärbung/photometrische Messungen)

2,1 %
2,0 %
1,8 %
2,4 %
2,1 %
2,0 %
**Mittelwert 2,066 %**

Johanniskrautöl/**Auszugsmittel Olivenöl** - % Hypericin (Intensität Rotfärbung/photometrische Messungen)

1,6 %
1,3 %
1,6 %
1,5 %
1,5 %
1,7 %
**Mittelwert 1,533 %.**

Aus diesen beiden Versuchen wird deutlich, daß die Ausbeute bezüglich der Farbintensität beim Extraktionsmittel Sojaöl um ca. 33 % höher liegt als beim Extraktionsmittel Olivenöl.

**Patentansprüche**

1. Verfahren zur Herstellung von Johanniskrautöl, bei dem Johanniskrautblüten mit einem Öl extrahiert werden, **gekennzeichnet durch** die folgenden Schritte:

   a) Vorbehandlung der Johanniskrautblüten, wobei die Vorbehandlung

      aa) ein Trocknen,
      ab) ein Befeuchten mit wäßriger alkoholischen Lösung umfaßt, wobei das Verhältnis des Gewichts der wäßrigen alkoholischen Lösung zum Trockengewicht der Johanniskrautblüten im Bereich von 3:1-1:1 liegt.

   b) Extraktion der Johanniskrautblüten in einem Ansatz mit mindestens einem Öl unter Rühren und/oder Schütteln, wobei die Extraktion in Schritt b) unter kontinuierlicher künstlicher Bestrahlung erfolgt.

2. Verfahren nach einem der vorangegangenen Ansprüche, **dadurch gekennzeichnet, daß** mindestens ein pflanzliches Öl verwendet wird, das ausgewählt ist aus der Gruppe, die umfasst: Sojaöl, Weizenkeimöl, Olivenöl, Sonnenblumenkernöl, Rapsöl, Leinöl, Kürbiskernöl, Sesamöl, Maiskeimöl, Palmkernöl, Distelöl und Erdnußöl.

3. Verfahren nach einem der vorangegangenen Ansprüche, **dadurch gekennzeichnet, daß** das Trocknen der Johanniskrautblüten bei 12-25°C erfolgt.

4. Verfahren nach einem der vorangegangenen Ansprüche, **dadurch gekennzeichnet, daß** das Befeuchten für 15 min - 10 h erfolgt.

5. Verfahren nach einem der vorangegangenen Ansprüche, **dadurch gekennzeichnet, daß** die kontinuierliche künstliche Bestrahlung im Spektralbereich von 400 nm - 710 nm erfolgt.

6. Verfahren nach einem der vorangegangenen Ansprüche, **dadurch gekennzeichnet, daß** die kontinuierliche künstliche Bestrahlung mit einer Lichtstromstärke von 800 Lumen - 2000 Lumen erfolgt.

7. Verfahren nach einem der vorangegangenen Ansprüche, **dadurch gekennzeichnet, daß** die Extraktion bei einer Temperatur im Bereich von 10 °C - 50 °C erfolgt.

8. Verfahren nach einem der vorangegangenen Ansprüche, **dadurch gekennzeichnet, daß** die Extraktion für 4 - 24 Tage erfolgt.

9. Verfahren nach einem der vorangegangenen Ansprüche, **gekennzeichnet durch** den weiteren Schritt:

   c) Aufbereiten des Ansatzes, wobei das Aufbereiten des Ansatzes die folgenden Teilschritte umfaßt:

      ca) Zugabe eines Trockenmittels unter Rühren und/oder Schütteln,
      cc) Filtrieren.

10. Verfahren nach einem der vorangegangenen Ansprüche, **dadurch gekennzeichnet, daß** das Verhältnis des Gewichts des Öls zum Trockengewicht der Johanniskrautblüten im Bereich von 22:1 bis 42:1 liegt.

11. Johanniskrautöl, herstellbar durch ein Verfahren nach einem der vorangegangenen Ansprüche, wobei als Öl zur Extraktion Sojaöl verwendet wird.

12. Johanniskrautöl nach Anspruch 11, **dadurch gekennzeichnet, daß** es eine Peroxidzahl aufweist, die im Bereich von 3-8 liegt.

13. Johanniskrautöl nach einem der Ansprüche 11 - 12, **dadurch gekennzeichnet, daß** es eine deutlich reduzierte Gefahr der Ranzilität aufweist.

**Claims**

1. Process for the preparation of St. John's wort oil in which St. John's wort flowers are extracted with an oil, **characterized by** the following steps:

   a) pre-treatment of the St. John's wort flowers, where the pre-treatment includes

      aa) a drying,
      ab) a moistening with aqueous alcoholic solution, where the ratio of the weight of the aqueous alcoholic solution to the dry weight of the St. John's wort flowers is in the range of 3:1-1:1.

   b) extraction of the St. John's wort flowers in a mixture with at least one oil with stirring and/or shaking, where the extraction in step b) takes place with continuous artificial irradiation.

2. Process according to any of the preceding claims, **characterized in that** at least one vegetable oil is used which is selected from the group including: soya oil, wheat germ oil, olive oil, sunflower seed oil, rapeseed oil, linseed oil, pumpkin seed oil, sesame oil, maize germ oil, palm kernel oil, safflower oil and peanut oil.

3. Process according to any of the preceding claims, **characterized in that** the drying of the St. John's wort flowers takes place at 12-25°C.

4. Process according to any of the preceding claims, **characterized in that** the moistening takes place for 15 min. - 10 h.

5. Process according to any of the preceding claims, **characterized in that** the continuous artificial irradiation takes place in the spectral range of 400 nm - 710 nm.

6. Process according to any of the preceding claims, **characterized in that** the continuous artificial irradiation takes place with a luminous flux of 800 lumen - 2000 lumen.

7. Process according to any of the preceding claims, **characterized in that** the extraction takes place at a temperature in the range of 10°C - 50°C.

8. Process according to any of the preceding claims, **characterized in that** the extraction takes place for 4 - 24 days.

9. Process according to any of the preceding claims, **characterized by** the further step:

   c) processing of the mixture, where the processing of the mixture includes the following subsidiary steps:

   ca) addition of a desiccant with stirring and/or shaking,
   cc) filtration.

10. Process according to any of the preceding claims, **characterized in that** the ratio of the weight of the oil to the dry weight of the St. John's wort flowers is in the range from 22:1 to 42:1.

11. St. John's wort oil which can be prepared by a process according to any of the preceding claims, where soya oil is used as oil for the extraction.

12. St. John's wort oil according to Claim 11, **characterized in that** it has a peroxide number which is in the range of 3-8.

13. St. John's wort oil according to any of Claims 11-12, **characterized in that** it has a distinctly reduced risk of rancility.

**Revendications**

1. Procédé de préparation d'huile de millepertuis, dans lequel des fleurs de millepertuis sont extraites avec une huile, **caractérisé par** les étapes suivantes :

   a) Prétraitement des fleurs de millepertuis, ledit prétraitement comprenant

   aa) un séchage,
   ab) un mouillage avec une solution alcoolique aqueuse, le rapport du poids de la solution alcoolique aqueuse sur le poids sec des fleurs de millepertuis se situant dans la plage de 3:1 à 1:1,

   b) Extraction des fleurs de millepertuis dans un mélange contenant au moins une huile en agitant et/ou en secouant, l'extraction de l'étape b) se faisant sous exposition continue à un rayonnement artificiel.

2. Procédé selon l'une des revendications précédentes, **caractérisé en ce que** l'on utilise au moins une huile végétale qui est choisie dans le groupe comprenant : l'huile de soja, l'huile de germes de blé, l'huile d'olive, l'huile de graines de tournesol, l'huile de colza, l'huile de lin, l'huile de pépins de courge, l'huile de sésame, l'huile de germes de maïs, l'huile de palme, l'huile de carthame et l'huile d'arachide.

3. Procédé selon l'une des revendications précédentes, **caractérisé en ce que** le séchage des fleurs de millepertuis se fait à une température de 12 à 25°C.

4. Procédé selon l'une des revendications précédentes, **caractérisé en ce que** le mouillage s'effectue pendant une durée de 15 minutes à 10 heures.

**5.** Procédé selon l'une des revendications précédentes, **caractérisé en ce que** l'exposition continue au rayonnement artificiel se fait dans le domaine spectral de 400 nm à 710 nm.

**6.** Procédé selon l'une des revendications précédentes, **caractérisé en ce que** l'exposition continue au rayonnement artificiel se fait avec une intensité de flux lumineux de 800 lumens à 2000 lumens.

**7.** Procédé selon l'une des revendications précédentes, **caractérisé en ce que** l'extraction se fait à une température dans la plage de 10°C à 50°C.

**8.** Procédé selon l'une des revendications précédentes, **caractérisé en ce que** l'extraction dure de 4 à 24 jours.

**9.** Procédé selon l'une des revendications précédentes, **caractérisé par** l'étape supplémentaire de :

c) Traitement du mélange, ledit traitement du mélange comprenant les étapes partielles suivantes :

ca) Ajout d'un agent dessiccatif en agitant et/ou en secouant,
cc) Filtration.

**10.** Procédé selon l'une des revendications précédentes, **caractérisé en ce que** le rapport du poids de l'huile sur le poids sec des fleurs de millepertuis se situe dans la plage de 22:1 à 42:1.

**11.** Huile de millepertuis pouvant être préparée par un procédé selon l'une des revendications précédentes, l'huile employée pour l'extraction étant de l'huile de soja.

**12.** Huile de millepertuis selon la revendication 11, **caractérisée en ce qu'**elle présente un indice de peroxyde qui se situe dans la plage de 3 à 8.

**13.** Huile de millepertuis selon la revendication 11 ou la revendication 12, **caractérisée en ce qu'**elle présente un risque de rancissement nettement réduit.